# EUROPEAN PATENT APPLICATION

(11) **EP 2 100 526 A1**
(43) Date of publication of application: **16.09.2009**
(21) Application number: 09002836.6
(22) Date of filing: 27.02.2009
(51) Int. Cl.: A41B 9/02, A41D 1/08, A61F 5/40

(54) **Men's trousers with genitals suspender**

(30) Priority: 03.03.2008 ES 200800448
(71) Applicant: Matamales Iscla, Pedro, 25700 La seu de Urgell - Lerida (ES); Zotta Baylo, Gianni, 25700 La Seu de Urgell - Lerida (ES); Dotti, Alessandro, 25700 La Seu de Urgell - Lerida (ES)
(72) Inventor: Matamales Iscla, Pedro, 25700 La seu de Urgell - Lerida (ES); Zotta Baylo, Gianni, 25700 La Seu de Urgell - Lerida (ES); Dotti, Alessandro, 25700 La Seu de Urgell - Lerida (ES)
(74) Representative: Isern-Jara, Jaime

(57) **Abstract**

Men's trousers with genitals' suspenders It comprises in its interior a "U"- shaped bag, linked by its front central part to the zone of the garment laying beneath the waist, whereas the side parts, provided in their edges with elastic waistbands each, define each a respective symmetrical side bag. The front and forward part of the trousers have centrally two vertical overlapping zones of the fabric, linked to the garment at alternate vertical sides, defining a vertical opening.

## Description

### Object of the Invention

The present invention relates to a pair of trousers for men with genitals' suspenders.

### Field of the Invention

The field of the invention is that of clothes and garment's manufacturing, especially that of men's underwear.

### Background of the Invention

The underwear clothing industry has put on the market, throughout the years, different kinds of underpants and also light trousers for internal or external use, for instance in this latter case, for playing sports and like activities.

Many attempts have been tried to house or fasten the masculine genital organs, but it must be said that such attempts have failed to succeed in that they didn't provide for the suitable positioning of all of the elements of the masculine genital organs. For playing sports and the like, a proper fastening and protection for such organs was missing, as well as the existence of a garment providing for the said functions.

### Brief description of the Invention

It is aimed to design a garment provided with a suspender device suited for the masculine genital organs, permitting at the same time complete freedom of movements and, of course, causing no inconvenience to the user during its use. Thus, the new trousers makes sure that those body parts are well positioned and can also be used for playing sports and to be a usual garment of a wardrobe for any activity of every-day's life, i.e. in the professional life.

To make the explanation easier, a set of drawings is attached to the present description, wherein it has been represented, by way of illustrating and no limiting example, an embodiment of a couple of trousers for men with genitals' suspender, according to the principles of the claims.

### Brief Description of the Drawings

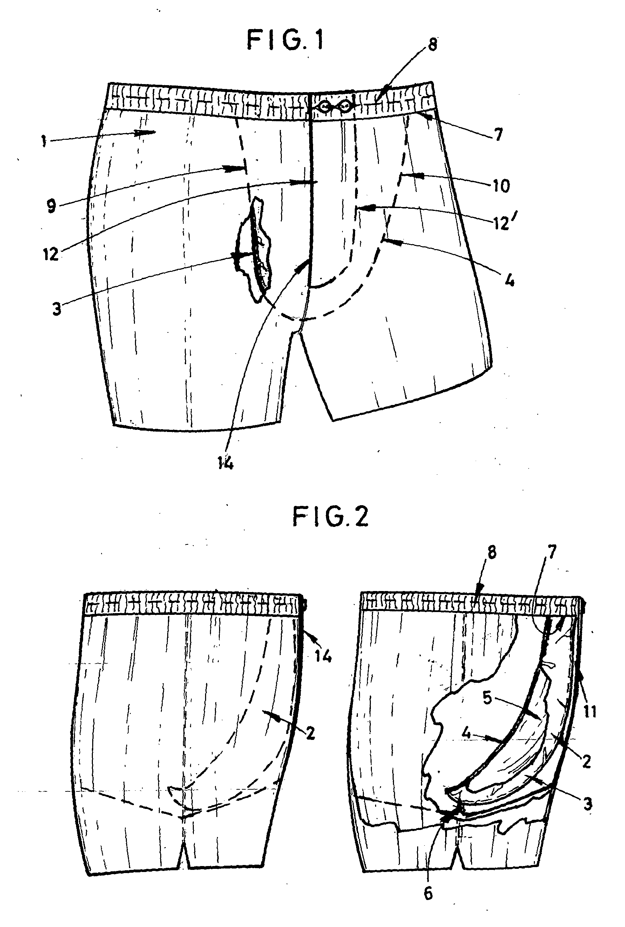

Figure 1 is a front external view of the new garment, where its structure is shown.
Figure 2 is an internal view of the same garment, where the configuration of the elements is shown

### Detailed Description of the INvention

The elements designated with numeral references correspond to the parts explained hereinafter.

The new pair of trousers comprises a body 1 intended both for external use, for example, for playing sports, and for internal use, with a suitable making and fabrics, for its use as underpants in the day-by-day life.

A feature of the present invention is the provision in its interior of a bag 2, preferably "U" shaped, intended for housing and fasten the masculine genital organs, permitting, of course, their natural movement as a consequence of the different positions of user's body, while retaining the said organs with freedom in a relatively reduced space. This increases the comfort for the user during the practice of conventional activities, avoiding the known disturbances caused by the uncontrolled friction of the men's genital parts with the fabric of the conventional trousers.

The bag 2 has a big "U"- shaped opening, defined by sides 3 and 4, advantageously adaptable as it comprises lengths of elastic band in each side, the lower central part 5 being attached by means of sewing to the internal part 6 of the trousers corresponding to the crotch. Thus there is defined in the lower part of bag 2 a bottom or space formed of two parts 9 and 10, corresponding to the natural shape of the masculine genital organs.

The upper front part 7 of the bag 2 is joined by means of sewing to the elastic waistband 8 usual in sport pants, in underpants and the likeThe internal front part of the bag 2, has also a second "U"-shaped elongated vertical opening 11, which opens to a zone 12 of the fabric forming the trousers' body 1, which overlaps with a zone 12' of the same fabric. Both overlapped zones define, in one of their opposite sides, conventionally, a vertical opening 14.

## Claims

1. Men's trousers with genitals' suspenders, **characterised in that** it comprises in its interior a "U"- shaped bag, linked by its front central part to the zone of the garment laying beneath the waist, whereas the side parts, provided in their edges with elastic waistbands each, define each a respective symmetrical side bag.

2. Men's trousers with genitals' suspenders, according to clam 1, **characterised in that** the front and forward part of the trousers have centrally two vertical overlapping zones of the fabric, linked to the garment at alternate vertical sides, defining a vertical opening.
